(19) Europäisches Patentamt / European Patent Office / Office européen des brevets

(11) **EP 3 474 952 B1**

(12) **EUROPEAN PATENT SPECIFICATION**

(45) Date of publication and mention
of the grant of the patent:
**28.07.2021 Bulletin 2021/30**

(21) Application number: **16751752.3**

(22) Date of filing: **03.08.2016**

(51) Int Cl.:
***A61Q 17/04*** *(2006.01)*     ***A61K 8/81*** *(2006.01)*
***A61K 8/02*** *(2006.01)*

(86) International application number:
**PCT/US2016/045328**

(87) International publication number:
**WO 2017/222570 (28.12.2017 Gazette 2017/52)**

(54) **COMPOSITIONS CONTAINING LATEX PARTICLES AND UV ABSORBERS**

ZUSAMMENSETZUNGEN MIT LATEXTEILCHEN UND UV-ABSORBERN

COMPOSITIONS CONTENANT DES PARTICULES DE LATEX ET DES ABSORBEURS UV

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **22.06.2016 US 201662353175 P**

(43) Date of publication of application:
**01.05.2019 Bulletin 2019/18**

(73) Proprietors:
• **Rohm and Haas Company**
**Collegeville, PA 19426 (US)**
• **Union Carbide Corporation**
**Seadrift, TX 77983 (US)**

(72) Inventors:
• **SHULMAN, Inna**
**Collegeville**
**PA 19426 (US)**
• **XU, Wenjun**
**Collegeville**
**PA 19426 (US)**
• **ZENG, Fanwen**
**Collegeville**
**PA 19426 (US)**

(74) Representative: **Houghton, Mark Phillip**
**Patent Outsourcing Limited**
**1 King Street**
**Bakewell, Derbyshire DE45 1DZ (GB)**

(56) References cited:
**EP-A2- 1 092 421**     **WO-A1-2010/118415**
**WO-A1-2016/028512**     **US-A- 5 663 213**

• **MCDONALD CHARLES J ET AL: "Hollow latex particles: synthesis and applications", ADVANCES IN COLLOID AND INTERFACE SCIENCE, XX, XX, vol. 99, no. 3, 2 December 2002 (2002-12-02), pages 181-213, XP002292602, DOI: 10.1016/S0001-8686(02)00034-9**

**Description**

**FIELD OF THE INVENTION**

This invention relates generally to personal care compositions comprising voided latex particles and UV radiation-absorbing agents.

**BACKGROUND**

Personal care compositions contain a variety of additives that provide a wide array of benefits to users. Sunscreen compositions in particular contain additives that offer protection from ultraviolet ("UV") radiation, which can damage the skin. UV radiation can be classified as UVA (long wave; i.e., wavelengths of 320-400 nm) and UVB (short wave; i.e., wavelengths of 290 to 320 nm). The efficacy of sunscreen formulations is measured by its sun protection factor ("SPF"). Since both UVA and UVB forms of radiation are harmful, sunscreen formulations offer protection from both kinds of rays. UV absorbing agents include physical blockers, such as titanium dioxide, and chemical absorbers, such as para-aminobenzoic acid and octyl methoxycinnamate. In certain compositions, it is desirable to decrease the level of UV absorbing agents due to undesirable aesthetic and toxicological effects.

To that end, personal care compositions comprising light scatterers and UV radiation-absorbing agents have been disclosed. For example, U.S. Patent No. 5,663,213 discloses a method of improving UV radiation absorption of a composition containing at least one UV radiation absorbing agent by incorporating a voided latex particle into the composition. Although the prior art discloses such particles for use in boosting the SPF of a composition in combination with a UV absorbing agent, such compositions are known to have elevated intensity of unpleasant odor profiles and are therefore not suitable for use in compositions such as face creams, lotions, an sunscreens.

Consequently, there is a need to develop new personal care compositions including UV absorbing agents and particle light scatterers, i.e., voided latex particles, that an SPF boosting effect while also providing a pleasing odor profile.

**STATEMENT OF INVENTION**

One aspect of the invention provides a personal care composition comprising (A) voided latex particles comprising (i) at least one core polymer comprising polymerized units derived from (a) 20 to 60 weight % of monoethylenically unsaturated monomers containing at least one carboxylic acid group, based on the total weight of the core polymer, and (b) 40 to 80 weight % of non-ionic ethylenically unsaturated monomers, based on the total weight of the core polymer, and (ii) at least one shell polymer comprising polymerized units derived from (a) 10 to 70 weight % of non-ionic ethylenically unsaturated monomers, based on the total weight of the shell polymer(s), and (b) 30 to 90 weight % of aliphatic monomers selected from the group consisting of allyl methacrylate, trimethylolpropane trimethacrylate, trimethylolpropane triacrylate, and combinations thereof, based on the total weight of the shell polymer(s), and (B) at least one UV absorbing agent, wherein the voided latex particles are present in an amount of from 0.5 to 20 weight %, based on the total weight of the composition, and wherein the voided latex particles contain a void and have a particle size of from 100 nm to 400 nm; and wherein an outermost shell comprises a shell polymer having polymerized units of the aliphatic monomers in an amount from 40 to 80 weight % based on the weight of the outermost shell polymer.

Another aspect of the invention provides a personal care composition as hereinbefore defined for use in a method for protecting skin from UV damage, the method comprising topically administering to the skin an effective amount of the personal care composition.

In another aspect, the invention provides a personal care composition as hereinbefore defined for use in a method for boosting the SPF or UV absorption of a sunscreen composition comprising adding to said sunscreen composition from 0.5 to 20 weight % of voided latex particles, based on the total weight of the composition.

**DETAILED DESCRIPTION**

The inventors have now surprisingly found that voided latex particles comprising a core polymer and at least one shell polymer provide SPF boosting and an improved odor profile, wherein the core polymer comprises polymerized units derived from monoethylenically unsaturated monomers containing at least one carboxylic acid group and non-ionic ethylenically unsaturated monomers, and the shell polymer comprises polymerized units derived from non-ionic ethylenically unsaturated monomers and aliphatic monomers selected from the group consisting of tri(meth)acrylates and (meth)acrylic monomers having mixed ethylenic functionality.

In the present invention, "personal care" is intended to refer to cosmetic and skin care compositions for leave on application to the skin including, for example, lotions, creams, gels, gel creams, serums, toners, wipes, masks, liquid foundations, make-ups, tinted moisturizer, oils, face/body sprays, topical medicines, and sunscreen compositions. "Sunscreen compositions" refers to compositions that protect the skin from UV damage. "Personal care" relates to compositions to be topically administered (i.e., not ingested). Preferably, the personal care composition is cosmetically acceptable. "Cosmetically acceptable" refers to ingredients typically used in personal care compositions, and is intended to underscore that materials that are toxic when present in the amounts typically found in personal care compositions are not contemplated as part of the present invention. The compositions of the invention may be manufactured by processes well known in the art, for example, by means of conventional mixing, dissolving, granulating, emulsifying, encapsulating, entrapping or lyophilizing processes.

As used herein, the term "polymer" refers to a polymeric compound prepared by polymerizing monomers, whether of the same or a different type. The generic term "polymer" includes the terms "homopolymer," "copolymer," and "terpolymer." As used herein, the term "polymerized units derived from" refers to polymer molecules that are synthesized according to polymerization techniques wherein a product polymer contains "polymerized units derived from" the constituent monomers which are the starting materials for the polymerization reactions. As used herein, the term "(meth)acrylic" refers to either acrylic or methacrylic.

As used herein, the terms "glass transition temperature" or "$T_g$" refers to the temperature at or above which a glassy polymer will undergo segmental motion of the polymer chain. Glass transition temperatures of a polymer can be estimated by the Fox equation (Bulletin of the American Physical Society, 1 (3) Page 123 (1956)) as follows:

$$1/T_g = w_1/T_{g(1)} + w_2/T_{g(2)}$$

For a copolymer, $w_1$ and $w_2$ refer to the weight fraction of the two comonomers, and $T_{g(1)}$ and $T_{g(2)}$ refer to the glass transition temperatures of the two corresponding homopolymers made from the monomers. For polymers containing three or more monomers, additional terms are added ($w_n/T_{g(n)}$). The $T_{(g)}$ of a polymer can also be calculated by using appropriate values for the glass transition temperatures of homopolymers, which may be found, for example, in "Polymer Handbook," edited by J. Brandrup and E.H. Immergut, Interscience Publishers. The $T_g$ of a polymer can also be measured by various techniques, including, for example, differential scanning calorimetry ("DSC"). The values of $T_g$ reported herein are measured by DSC.

The inventive personal care compositions contain voided latex particles. Voided latex particles useful in the invention comprise a multistaged particle containing at least one core polymer and at least one shell polymer. The ratio of the core weight to the total polymer weight is from 1:4 (25% core) to 1:100 (1% core), and preferably from 1:8 (12% core) to 1:50 (2% core).

The at least one core polymer includes polymerized units derived from monoethylenically unsaturated monomers containing at least one carboxylic acid group, and non-ionic ethylenically unsaturated monomers. The core polymer may be obtained, for example, by the emulsion homopolymerization of the monoethylenically unsaturated monomer containing at least one carboxylic acid group or by copolymerization of two or more of the monoethylenically unsaturated monomers containing at least one carboxylic acid group. In certain embodiments, the monoethylenically unsaturated monomer containing at least one carboxylic acid group is copolymerized with one or more non-ionic (that is, having no ionizable group) ethylenically unsaturated monomers. While not wishing to be bound by theory, it is believed that the presence of the ionizable acid group makes the core swellable by the action of a swelling agent, such as an aqueous or gaseous medium containing a base to partially neutralize the acid core polymer and cause swelling by hydration.

Suitable monoethylenically unsaturated monomers containing at least one carboxylic acid group of the core polymer include, for example, (meth)acrylic acid, (meth)acryloxypropionic acid, itaconic acid, aconitic acid, maleic acid, fumaric acid, crotonic acid, citraconic acid, maleic anhydride, monomethyl maleate, monomethyl fumarate, and monomethyl itaconate, and other derivatives such as corresponding anhydride, amides, and esters. In certain preferred embodiments, the monoethylenically unsaturated monomers containing at least one carboxylic acid group are selected from acrylic acid and methacrylic acid. In certain embodiments, the core comprises polymerized units of monoethylenically unsaturated monomers containing at least one carboxylic acid group in an amount of from 20 to 60 weight %, preferably from 30 to 50 weight %, and more preferably from 35 to 45 weight %, based on the total weight of the core polymer.

Suitable non-ionic ethylenically unsaturated monomers of the core polymer include, for example, styrene, vinyltoluene, ethylene, vinyl acetate, vinyl chloride, vinylidene chloride, acrylonitrile, (meth)acrylamide, ($C_1$-$C_{22}$)alkyl and ($C_3$-$C_{20}$)alkenyl esters of (meth)acrylic acid, such as methyl (meth)acrylate, ethyl (meth)acrylate, butyl (meth)acrylate, 2-ethylhexyl (meth)acrylate, benZyl (meth)acrylate, lauryl (meth)acrylate, oleyl (meth)acrylate, palmityl (meth)acrylate and stearyl (meth)acrylate. In certain preferred embodiments, the non-ionic ethylenically unsaturated monomers are selected from methyl methacrylate and butyl methacrylate. In certain embodiments, the core comprises polymerized units of non-ionic ethylenically unsaturated monomers in an amount of from 40 to 80 weight %, preferably from 50 to 70 weight %, and more preferably from 55 to 65 weight %, based on the total weight of the core polymer.

The voided latex particles suitable for use in the present invention also include at least one shell polymer. The at least one shell polymer(s) comprise polymerized units derived from non-ionic ethylenically unsaturated monomers and polyethylenically unsaturated monomers. In certain embodiments, at least one shell polymer optionally comprises polymerized units derived from at least one of monoethylenically unsaturated monomers containing at least one carboxylic acid group and monoethylenically unsaturated monomers containing at least one "non-carboxylic" acid group. In certain embodiments, the shell portion of the voided latex particles are polymerized in a single stage, preferably in two stages, and more preferably in at least three stages. As used herein, the term "outermost shell" refers to the composition of the final distinct polymerization stage used to prepare the voided latex particles. In certain embodiments wherein the outermost shell is provided by a multistage polymerization process, the outermost shell comprises at least 25 weight %, preferably at least 35 weight %, and more preferably at least 45 weight % of the total shell portion of the voided latex particle.

Suitable non-ionic ethylenically unsaturated monomers for the at least one shell polymer include, for example, vinyl acetate, acrylonitrile, methacrylonitrile, nitrogen containing ring compound unsaturated monomers, vinylaromatic monomers, ethylenic monomers and selected (meth)acrylic acid derivatives. Suitable (meth)acrylic acid derivatives include, for example, $(C_1-C_{22})$alkyl (meth)acrylate, substituted (meth)acrylate, and substituted (meth)acrylamide monomers. In certain preferred embodiments, the (meth)acrylic acid derivatives are selected from methyl acrylate, methyl methacrylate, ethyl acrylate, ethyl methacrylate, butyl acrylate, butyl methacrylate, isobutyl acrylate, isobutyl methacrylate, hydroxyethyl methacrylate, hydroxypropyl methacrylate, dimethylaminoethyl methacrylate, dimethylaminopropyl methacrylamide, and mixtures thereof. Suitable vinylaromatic monomers include, for example, styrene, $\alpha$-methylstyrene, vinyltoluene, alkyl-substititued styrene (such as t-butylstyrene and ethylvinylbenzene), and halogenated styrenes (such as chlorostyrene and 3,5-bis (trifuoromethyl)styrene). In certain preferred embodiments, the non-ionic ethylenically unsaturated monomers comprise at least one of styrene, ethylvinylbenzene, t-butylstrene, and mixtures thereof. In certain preferred embodiments, the non-ionic ethylenically unsaturated monomers comprise styrene. In certain embodiments, the outermost shell polymer comprises polymerized units of non-ionic ethylenically unsaturated monomers in an amount of from 10 to 70 weight %, preferably from 15 to 60 weight %, and more preferably from 20 to 50 weight %, based on the total weight of the outermost shell polymer.

The aliphatic monomers comprise at least one of trimethylolpropane trimethacrylate, trimethylolpropane triacrylate. In certain preferred embodiments, the tri(meth)acrylate monomers comprise trimethylolpropane trimethacrylate. The outermost shell comprises polymerized units of aliphatic monomers in an amount of from 40 to 80 weight %, based on the weight of the outermost shell polymer.

Suitable monoethylenically unsaturated monomers containing at least one carboxylic acid group for the shell polymer(s) include, for example, (meth)acrylic acid, (meth)acryloxypropionic acid, itaconic acid, aconitic acid, maleic acid, fumaric acid, crotonic acid, citraconic acid, maleic anhydride monomethyl maleate, monomethyl fumarate, and monomethyl itaconate, and other derivatives such as corresponding anhydride, amides, and esters. In certain preferred embodiments, the monoethylenically unsaturated monomers containing at least one carboxylic acid group are selected from acrylic acid and methacrylic acid. In certain embodiments, the shell polymer(s) comprises polymerized units of monoethylenically unsaturated monomers containing at least one carboxylic acid group in an amount of from 0.1 to 10 weight %, preferably from 0.3 to 7.5 weight %, and more preferably from 0.5 to 5 weight %, based on the total weight of the shell polymer(s).

Suitable monoethylenically unsaturated monomers containing at least one "non-carboxylic" acid group for the shell polymer(s) include, for example, allylsulfonic acid, allylphosphonic acid, allyloxybenzenesulfonic acid, 2-acrylamido-2-methylpropanesulfonic acid (the acryonym "AMPS" for this monomer is a trademark of Lubrizol Corporation, Wickliffe, Ohio, USA), 2-hydroxy-3-(2-propenyloxy)propanesulfonic acid, 2-methyl-2-propene-1-sulfonic acid, 2-methacrylamido-2-methyl-1-propanesulfonic acid, 3-methacrylamido-2-hydroxy-1-propanesulfonic acid, 3-sulfopropyl acrylate, 3-sulfopropyl methacrylate, isopropenylphosphonic acid, vinylphosphonic acid, phosphoethyl methacrylate, styrenesulfonic acid, vinylsulfonic, acid and the alkali metal and ammonium salts thereof. In certain preferred embodiments, the monoethylenically unsaturated monomers containing at least one "non-carboxylic" acid group are selected from 2-acrylamido-2-methylpropanesulfonic acid, styrenesulfonic acid, and sodium styrene sulfonate. In certain embodiments, the shell polymer(s) comprise polymerized units of monoethylenically unsaturated monomers containing at least one "non-carboxylic" acid group in an amount of from 0.1 to 10 weight %, preferably from 0.5 to 7.5 weight %, and more preferably from 1 to 5 weight %, based on the total weight of the shell polymer(s).

The shell polymer(s) of the latex particles suitable for use in the present invention have $T_g$ values which are high enough to support to support the void within the latex particle. In certain embodiments, the $T_g$ values of at least one shell are greater than 50°C , preferably greater than 60°C, and more preferably greater than 70°C.

In certain embodiments, the core polymer and shell polymer are made in a single polymerization step. In certain other embodiments, the core polymer and shell polymer are made in a sequence of polymerization steps. Suitable polymerization techniques for preparing the voided latex particles contained in the inventive personal care compositions include, for example, sequential emulsion polymerization. In certain embodiments, the monomers used in the emulsion polymerization of the shell polymer of the voided latex particles comprise one or more non-ionic ethylenically unsaturated monomer. Aqueous emulsion polymerization processes typically are conducted in an aqueous reaction mixture, which contains at least one monomer and various synthesis adjuvants, such as the free radical sources, buffers, and reductants in an aqueous reaction medium. In certain embodiments, a chain transfer agent may be used to limit molecular weight. The aqueous reaction medium is the continuous fluid phase of the aqueous reaction mixture and contains more than 50 weight % water and optionally one or more water miscible solvents, based on the weight of the aqueous reaction medium. Suitable water miscible solvents include, for example, methanol, ethanol, propanol, acetone, ethylene glycol ethyl ethers, propylene glycol propyl ethers, and diacetone alcohol.

In certain embodiments, the void of the latex particles is prepared by swelling the core with a swelling agent containing one or more volatile components. The swelling agent permeates the shell to swell the core. The volatile components of the swelling agent can then be removed by drying the latex particles, causing a void to be formed within the latex particles. In certain embodiments, the swelling agent is an aqueous base. Suitable aqueous bases useful for swelling the core include, for example, ammonia, ammonium hydroxide, alkali metal hydroxides, such as sodium hydroxide, or a volatile amine such as trimethylamine or triethylamine. In certain embodiments, the voided latex particles are added to the composition with the swelling agent present in the core. When the latex particles are added to the composition with the swelling agent present in the core, the volatile components of the swelling agent will be removed upon drying of the composition. In certain other embodiments, the voided latex particles are added to the composition after removing the volatile components of the swelling agent.

In certain embodiments, the voided latex particles contain a void with a void fraction of from 1% to 70%, preferably from 5% to 50%, more preferably from 10% to 40%, and even more preferably from 25% to 35%. The void fractions are determined by comparing the volume occupied by the latex particles after they have been compacted from a dilute dispersion in a centrifuge to the volume of non-voided particles of the same composition. In certain embodiments, the voided latex particles have a particle size of from 100 nm to 400 nm, preferably from 150 nm to 375 nm, and more preferably from 190 nm to 350 nm, as measured by a Brookhaven BI-90.

A person of ordinary skill in the art can readily determine the effective amount of the voided latex particles that should be used in a particular composition in order to provide the benefits described herein (e.g., maintained UV absorption while providing a more pleasing odor profile when applied to skin), via a combination of general knowledge of the applicable field as well as routine experimentation where needed. By way of non-limiting example, the amount of voided latex particles in the composition of the invention may be in the range of from 0.5 to 20 solids weight %, preferably from 1 to 10 solids weight %, more preferably from 1 to 5 solids weight %, based on the total weight of the composition.

The personal care compositions of the present invention also comprise at least one UV absorbing agent. Suitable UV absorbing agents include, for example, oxybenzone, dioxybenzone, sulisobenzone, menthyl anthranilate, para-aminobenzoic acid, amyl paradimethylaminobenzoic acid, octyl para-dimethylaminobenzoate, ethyl 4-bis (hydroxypropyl) para-aminobenzoate, polyethylene glycol (PEG-25) para-aminobenzoate, ethyl 4-bis (hydroxypropyl) aminobenzoate, diethanolamine para-methyoxycinnamate, 2-ethoxyethyl para-methoxycinnamate, ethylhexyl para-methoxycinnamate, octyl para-methoxycinnamate, isoamyl para-methoxycinnamate, 2-ethylhexyl-2-cyano-3,3-diphenyl-acrylate, 2-ethylhexyl salicylate, homomenthyl salicylate, glyceryl aminobenzoate, triethanolamine salicylate, digalloyl trioleate, lawsone with dihydroxyacetone, 2-phenylbenzimidazole-5-sulfonic acid, 4-methylbenzylidine camphor, avobenzone, titanium dioxide, and zinc oxide. Alternatively, UV absorbing agents such as triazines, benzotriazoles, vinyl group-containing amides, cinnamic acid amides and sulfonated benzimidazoles may also be used. In certain embodiments, the personal care compositions include UV absorbing agents in an amount of from 0.1 to 50 weight %, preferably 5 to 40 weight %, and more preferably 10 to 30 weight %, based on the total weight of the composition.

Compositions of the invention also include a dermatologically acceptable carrier. Such material is typically characterized as a carrier or a diluent that does not cause significant irritation to the skin and does not negate the activity and properties of active agent(s) in the composition. Examples of dermatologically acceptable carriers that are useful in the invention include, without limitation, water, such as deionized or distilled water, emulsions, such as oil-in-water or water-in-oil emulsions, alcohols, such as ethanol, isopropanol or the like, glycols, such as propylene glycol, glycerin or the like, creams, aqueous solutions, oils, ointments, pastes, gels, lotions, milks, foams, suspensions, powders, or mixtures thereof. In some embodiments, the composition contains from about 99.99 to about 50 percent by weight of the dermatologically acceptable carrier, based on the total weight of the composition.

The personal care composition of the invention may also include, for instance, a thickener, additional emollients, an emulsifier, a humectant, a surfactant, a suspending agent, a film forming agent, a lower monoalcoholic polyol, a high boiling point solvent, a propellant, a mineral oil, silicon feel modifiers, or mixtures thereof. The amount of optional ingredients effective for achieving the desired property provided by such ingredients can be readily determined by one skilled in the art.

Other additives may be included in the compositions of the invention such as, but not limited to, abrasives, absorbents, aesthetic components such as fragrances, pigments, colorings/colorants, essential oils, skin sensates, astringents (e.g., clove oil, menthol, camphor, eucalyptus oil, eugenol, menthyl lactate, witch hazel distillate), preservatives, anti-caking agents, a foam building agent, antifoaming agents, antimicrobial agents (e.g., iodopropyl butylcarbamate), antioxidants, binders, biological additives, buffering agents, bulking agents, chelating agents, chemical additives, colorants, cosmetic astringents, cosmetic biocides, denaturants, drug astringents, external analgesics, film formers or materials, e.g., polymers, for aiding the film-forming properties and substantivity of the composition (e.g., copolymer of eicosene and vinyl pyrrolidone), opacifying agents, pH adjusters, propellants, reducing agents, sequestrants, skin bleaching and lightening agents (e.g., hydroquinone, kojic acid, ascorbic acid, magnesium ascorbyl phosphate, ascorbyl glucosamine), skin-conditioning agents (e.g., humectants, including miscellaneous and occlusive), skin soothing and/or healing agents (e.g., panthenol and derivatives (e.g., ethyl panthenol), aloe vera, pantothenic acid and its derivatives, allantoin, bisabolol, and dipotassium glycyrrhizinate), skin treating agents, and vitamins (e.g., Vitamin C) and derivatives thereof. The amount of option ingredients effective for achieving the desired property provided by such ingredients can be readily determined by one skilled in the art.

As noted above, skin care compositions of the present invention are highly effective as SPF and UV absorption boosters. Accordingly, the skin care compositions of the present invention are useful for the treatment and protection of skin, including, for example, protection from UV damage, moisturization of the skin, prevention and treatment of dry skin, protection of sensitive skin, improvement of skin tone and texture, masking imperfections, and inhibition of trans-epidermal water loss. Thus, in one aspect the present invention provides that the personal care compositions may be used in a method for protecting skin from UV damage comprising topically administering to the skin a composition comprising (a) 0.1 to 50 weight % inorganic metal oxide particles, based on the weight of the composition, and (b) 0.5 to 50 weight % of a UV absorbing agent, based on the weight of the composition. The compositions may also be used in a method for boosting the SPF or UV absorption of a sunscreen composition containing a UV absorbing agent and the voided latex particles as described herein.

In practicing the methods of the invention, the skin care compositions are generally administered topically by applying or spreading the compositions onto the skin. A person of ordinary skill in the art can readily determine the frequency with which the compositions should be applied. The frequency may depend, for example, on the level of exposure to UV light that an individual is likely to encounter in a given day and/or the sensitivity of the individual to UV light. By way of non-limiting example, administration on a frequency of at least once per day may be desirable.

Some embodiments of the invention will now be described in detail in the following Examples.

## EXAMPLES

### Example 1

#### *Preparation of Exemplary and Comparative Copolymer Particles*

Exemplary voided latex particles according to the present invention and comparative particles contain a core, first shell, second shell, and third shell (i.e., the "outermost" shell), in the amount of 4.7 weight %, 22.1 weight %, 26.8 weight %, and 46.4 weight %, respectively, by total weight of the particles. The exemplary and comparative particles all contain the same monomer composition of the core, first shell, and second shell, as recited in Table 1.

**Table 1. Core, First Shell, and Second Shell of Exemplary and Comparative Particles**

| Monomer (wt %) | |
|---|---|
| Core (4.7%): | 60 MMA / 40 MAA |
| Shell 1 (22.1%): | 8.5 BMA / 88.5 MMA / 3 MAA |
| Shell 2 (26.8%): | 94.9 Sty / 5.1 DVB |
| MMA = methyl methacrylate<br>MAA = methacrylic acid<br>BMA = butyl methacrylate<br>Sty = styrene<br>DVB = divinylbenzene | |

The composition of the third shell (i.e., the "outermost" shell) of the exemplary and comparative particles contain the monomer compositions recited in Table 2.

**Table 2. Third (Outermost) Shell of Exemplary and Comparative Particles**

| Sample | Monomer (wt %) |
|---|---|
| P-E1 | Shell 3 (46.4 wt %): 22.3 MMA / 75 ALMA / 2.7 SSS |
| P-E2 | Shell 3 (46.4 wt %): 47.3 MMA / 50 ALMA / 2.7 SSS |

(continued)

| Sample | Monomer (wt %) |
|--------|----------------|
| P-E3 | Shell 3 (46.4 wt %): 62.3 MMA / 35 ALMA / 2.7 SSS |
| P-E4 | Shell 3 (46.4 wt %): 22.3 Sty / 75 ALMA / 2.7 SSS |
| P-E5 | Shell 3 (46.4 wt %): 43.7 Sty / 35 ALMA / 15 TMPTMA / 2.7 SSS |
| P-E6 | Shell 3 (46.4 wt %): 38.9 Sty / 9 MMA / 50 ALMA / 2.7 SSS |
| P-C1* | Shell 3 (46.4 wt %): 46.2 Sty / 51.1 DVB / 2.7 SSS |
| P-C2* | Shell 3 (46.4 wt %): 62.3 Sty / 35 DVB / 2.7 SSS |
| P-C3* | Shell 3 (46.4 wt %): 77.3 MMA / 20 ALMA / 2.7 SSS |
| P-C4* | Shell 3 (46.4 wt %): 93.3 Sty / 4 ALMA / 2.7 SSS |

MMA = methyl methacrylate
MAA = methacrylic acid
BMA = butyl methacrylate
Sty = styrene
TMPTMA = trimethylolpropane trimethacrylate
SSS = sodium styrene sulfonate
*Comparative

For exemplary voided latex particle P-E1, 875.3 grams (g) deionized water was added to a 3-liter, 4-neck round bottom flask equipped with overhead stirrer, thermocouple, heating mantle, adapter inlet, Claisen head fitted with a water condenser and nitrogen inlet, and heated to 84°C under nitrogen. To the heated water was added 0.30 g acetic acid, 1.70 g sodium persulfate in 15.5 g of deionized water followed by the addition of an aqueous dispersion of 31% poly(MMA/MAA//60/40) acrylic seed (core) polymer, having an average particle diameter of approximately 110 to 220 nm. To this heated mixture at 82° C, a monomer emulsion containing 71.5 g deionized water, 2.1 g aqueous solution of 23% SDBS, 91.6 g MMA, 8.9 g BMA and 3.1 g MAA was metered in over 90 minutes followed by a deionized water rinse. Next, a solution of 0.65 g sodium persulfate in 32.8 g deionized water was added over 90 minutes and the reaction temperature was raised to 90°C concurrent with the addition of a second monomer emulsion containing 48.3 g deionized water, 0.35 g aqueous solution of 23% SDBS, 120.5 g Sty, 6.45 g DVB and 0.70 g linseed oil fatty acid over 30 minutes. At the completion of addition of the second monomer emulsion, 8.0 g aqueous 28% ammonium hydroxide was added, and hold for 10 min. To the reaction mixture at 91°C was added, over 60 minutes, a third monomer emulsion containing 100.5 g deionized water, 1.0 g aqueous solution of 23% SDBS, 50.3 g ALMA, and 6.1 g of sodium styrene sulfonate, followed by a deionized water rinse. The reactor contents were held at 91°C for 30 minutes, then 5.8 g of aqueous solution containing 0.10 g of FeSO4.7H2O and 0.10 g of versene was added followed by the concurrent addition over 60 minutes of 5.10 g of t-butylhydrogen peroxide (70%) in 19.0 g of deionized water and 2.6 g isoascorbic acid in 19.0 g deionized water, to the reactor maintained at 91°C. The latex was cooled to room temperature and then filtered.

All other exemplary and comparative particles were prepared substantially as described above, with the appropriate changes in monomer amounts as recited in Table 2.

**Example 2**

**_Characterization of Exemplary and Comparative Latex Particles_**

Voided latex particles as prepared in Example 1 were evaluated for particle size and percent void fraction, as shown in Table 3.

**Table 3. Characterization of Latex Particles**

| Sample | Particle Size (nm) | % Void Fraction |
|--------|--------------------|-----------------|
| P-E1 | 359 | 23.4 |
| P-E2 | 354 | 26.2 |
| P-E3 | 360 | 27.2 |

(continued)

| Sample | Particle Size (nm) | % Void Fraction |
|---|---|---|
| P-E4 | 356 | 26.3 |
| P-E5 | 350 | 27.2 |
| P-E6 | 340 | 27.0 |
| P-C1* | 320 | 30.7 |
| P-C2* | 362 | 30 |
| P-C3* | 285 | 28.9 |
| P-C4* | 346 | 25.8 |
| * Comparative | | |

The particle size was measured using a Brookhaven BI-90. The percent void fraction of the latex particles was measured by making a 10% by weight dispersion of each sample with propylene glycol, which was then mixed and poured into a weight-per-gallon cup which was capped and weighed. A 10 % water blank was also measured, and the difference in the weight was used to calculate the density of the sample, from which the percent void fraction was determined.

**Example 3**

***Preparation of Exemplary Sunscreen Formulations***

Exemplary sunscreen formulations according to the present invention contain the components recited in Table 4.

**Table 4. Exemplary Sunscreen Formulations**

| Trade Name | INCI | S-E1 (pbw) | S-E2 (pbw) | S-E3 (pbw) | S-E4 (pbw) | S-E5 (pbw) | S-E6 (pbw) |
|---|---|---|---|---|---|---|---|
| **Phase A** | | | | | | | |
| -- | DI Water | q.s. to 100 | q.s. to 100 | q.s. to 100 | q.s. to 100 | q.s. to 100 | q.s. to 100 |
| ACULYN 33[1] | Acrylates copolymer | 3.33 | 3.33 | 3.33 | 3.33 | 3.33 | 3.33 |
| -- | Glycerin | 1.00 | 1.00 | 1.00 | 1.00 | 1.00 | 1.00 |
| EDTA | Ethylenediaminetetraacetic acid tetras odium salt | 0.10 | 0.10 | 0.10 | 0.10 | 0.10 | 0.10 |
| **Phase B** | | | | | | | |
| Escalol 557[2] | Octyl methoxycinnamate | 6.00 | 6.00 | 6.00 | 6.00 | 6.00 | 6.00 |
| Escalol 567[2] | Benzophenone-3 | 2.00 | 2.00 | 2.00 | 2.00 | 2.00 | 2.00 |
| Ceraphyl 41[2] | $(C_{12}$-$C_{15})$alkyl lactate | 2.00 | 2.00 | 2.00 | 2.00 | 2.00 | 2.00 |
| EPITEX 66[1] | Acrylates copolymer | 1.50 | 1.50 | 1.50 | 1.50 | 1.50 | 1.50 |
| Dow Corning 345 Fluid[3] | Cylco-methicone | 2.00 | 2.00 | 2.00 | 2.00 | 2.00 | 2.00 |
| -- | Stearic acid | 1.50 | 1.50 | 1.50 | 1.50 | 1.50 | 1.50 |
| **Phase C** | | | | | | | |
| -- | Triethanolamine | 0.85 | 0.85 | 0.85 | 0.85 | 0.85 | 0.85 |
| **Phase D** | | | | | | | |
| P-E1 | -- | 5.00 (solids) | -- | -- | -- | -- | -- |

(continued)

| Phase D | | | | | | | |
| --- | --- | --- | --- | --- | --- | --- | --- |
| P-E2 | -- | -- | 5.00 (solids) | -- | -- | -- | -- |
| P-E3 | -- | -- | -- | 5.00 (solids) | -- | -- | -- |
| P-E4 | -- | -- | -- | -- | 5.00 (solids) | -- | -- |
| P-E5 | -- | -- | -- | -- | -- | 5.00 (solids) | -- |
| P-E6 | -- | -- | -- | -- | -- | -- | 5.00 (solids) |
| Total | | 100 | 100 | 100 | 100 | 100 | 100 |
| [1]Available from The Dow Chemical Company<br>[2]Available from International Specialty Products<br>[3] Available from Dow Corning | | | | | | | |

The exemplary sunscreen formulations were prepared by mixing Phase A components and heating to 75°C. In a separate vessel Phase B components were mixed together and heated to 75° C. With adequate agitation, Phase B was mixed into Phase A. After complete mixing, Phase C was added to the A/B mixture and the mixture was then cooled to 40°C, while maintaining agitation. When the mixture was 40°C or lower, Phase D (latex particles) was added as dispersion, having been prepared by emulsion polymerization. A control composition, hereinafter referred to as "Control," was also prepared according to the composition as shown in Table 3, except that no latex polymer particles were added. The acrylates copolymer (as ACULYN 33) was added to the composition to provide thickening; glycerin was added as a humectant; tetrasodium EDTA (ethylenediamine tetraacetic acetate) was added for mineral ion control; octylmethoxycinnamate and benzophenone-3 (as Escalol 557 and Escalol 567, respectively) were added as UV radiation-absorbing agents; $(C_{12}\text{-}C_{15})$alkyl lactate (as Ceraphyl 41) was added as an emollient and excipient; acrylates copolymer (as Epitex 66) was added as a waterproofing agent and a film-former; cyclomethicone (as Dow Corning 345 Fluid) was added as an emollient and excipient; stearic acid was added as the emulsifier; and triethanolamine was added as a neutralizing agent for both the stearic acid and the acrylates copolymer.

## Example 4

### Preparation of Comparative Sunscreen Formulations

Comparative sunscreen formulations according to the present invention contain the components recited in Table 5.

#### Table 5. Comparative Sunscreen Formulations

| Trade Name | INCI | S-C1 (pbw) | S-C2 (pbw) | S-C3 (pbw) | S-C4 (pbw) |
| --- | --- | --- | --- | --- | --- |
| Phase A | | | | | |
| -- | DI Water | q.s. to 100 | q.s. to 100 | q.s. to 100 | q.s. to 100 |
| ACULYN 33[1] | Acrylates copolymer | 3.33 | 3.33 | 3.33 | 3.33 |
| -- | Glycerin | 1.00 | 1.00 | 1.00 | 1.00 |
| EDTA | Ethylenediaminetetraacetic acid tetras odium salt | 0.10 | 0.10 | 0.10 | 0.10 |
| Phase B | | | | | |
| Escalol 557[2] | Octyl methoxycinnamate | 6.00 | 6.00 | 6.00 | 6.00 |
| Escalol 567[2] | Benzophenone-3 | 2.00 | 2.00 | 2.00 | 2.00 |

(continued)

| Phase B | | | | | |
|---------|---|---|---|---|---|
| Ceraphyl 41[2] | $(C_{12}\text{-}C_{15})$alkyl lactate | 2.00 | 2.00 | 2.00 | 2.00 |
| EPITEX 66[1] | Acrylates copolymer | 1.50 | 1.50 | 1.50 | 1.50 |
| Dow Corning 345 Fluid[3] | Cylco-methicone | 2.00 | 2.00 | 2.00 | 2.00 |
| -- | Stearic acid | 1.50 | 1.50 | 1.50 | 1.50 |
| Phase C | | | | | |
| -- | Triethanolamine | 0.85 | 0.85 | 0.85 | 0.85 |
| Phase D | | | | | |
| P-C1 | -- | 5.00 (solids) | -- | -- | -- |
| P-C2 | -- | -- | 5.00 (solids) | -- | -- |
| P-C3 | -- | -- | -- | 5.00 (solids) | -- |
| P-C4 | -- | -- | -- | -- | 5.00 (solids) |
| | Total | 100 | 100 | 100 | 100 |
| [1]Available from The Dow Chemical Company [2]Available from International Specialty Products [3] Available from Dow Corning | | | | | |

The comparative sunscreen formulations were prepared substantially as described in Example 3.

**Example 5**

***SPF Boost Heat Aging Study of Exemplary and Comparative Sunscreen Formulations***

Exemplary and comparative sunscreen formulations as prepared in Examples 3 and 4 were evaluated for the capacity to retain the ability to absorb UV radiation after heat aging by measuring the sun protection factor (SPF) of the test formulations. The SPF was measured using a UV-2000S with an integrating sphere and SPF Operating Software supplied by LabSpheres (North Sutton, NH, USA). The UV-2000S measures the UV absorbance of a sample over UV radiation Wavelengths (290-400 nm for each sample) and calculates an SPF value based on this UV absorbance spectrum. The following procedure for measuring SPF was used.

The compositions prepared were coated at a level of 7 milligram, on a 5cm by 5cm PMMA plate using a wire round rod. The SPF values were measured initially, after 4 weeks of storage at 45°C, and after 8 weeks of storage of the formulated samples at 45°C. The "Control" was also measured and stored in the same manner. The SPF Boost Factor (SBF) values were calculated as follows:

$$SBF = \frac{SPF_s - SPF_c}{SPF_c} \times 100\%$$

where $SPF_s$ is the measured SPF value of the "sample" at a given time (e.g., 4 weeks or 8 weeks) and at a given storage temperature (either room temperature or 45°C), $SPF_c$ is the measured SPF value of the "control" at the same time and at the same given storage temperature with $SPF_s$.

The accelerated aging tests described herein are believed to approximate the expected shelf-life for commercial formulations (containing latex particles of the present invention) stored at ambient temperatures: for example, 2 weeks at 45°C is an estimate of shelf-life after 3 months, 4 weeks at 45°C is an estimate of shelf-life after 6 months, 3 months at 45°C is an estimate of shelf-life after 1.5 years.

The SPF Boost Retention (SBR) after certain period of heat age of the voided latex particles are therefore calculated as follows:

$$SBR = \frac{SBF_{ha}}{SBF_i}$$

where $SBF_{ha}$ is the SPF boost factor of the "sample" at a given heat age period of time (e.g., 1 week, 4 weeks, or 8 weeks at 45°C), and $SBF_i$ is the initial SPF boost factor of the "sample" without heat age.

The SPF retention after heat age provided by the crosslinked shell polymer composition is represented as: maintained (when SBR $\geq$ 0.8), partially maintained (0.2 < SBR < 0.8), and not maintained (SBR $\leq$ 0.2). For samples that partially or did not maintain SBR after 4 weeks of storage at 45°C, heat-aging studies for 8 weeks were not performed. The results of the SBR study are shown in Table 6.

**Table 6. SPF Boost Retention of Exemplary and Comparative Sunscreen Formulations**

| Sample | SBR at 4 weeks (45°C) | SBR at 8 weeks (45°C) |
|---|---|---|
| S-E1 | 1.3 (maintained) | 1.79 (maintained) |
| S-E2 | 0.87 (maintained) | 1.16 (maintained) |
| S-E3 | 0.6 (partially maintained) | -- |
| S-E4 | 0.86 (maintained) | 1.14 (maintained) |
| S-E5 | 1.13 (maintained) | 0.79 (partially maintained) |
| S-E6 | 0.8 (partially maintained) | 0.75 (partially maintained) |
| S-C1* | 1.1 (maintained) | 1.65 (maintained) |
| S-C2* | 1.75 (maintained) | 1.84 (maintained) |
| S-C3* | 0.6 (partially maintained) | -- |
| S-C4* | 0 (not maintained) | -- |
| *Comparative | | |

The results demonstrate that exemplary sunscreen formulations prepared according to the present invention provide an SPF Boost Retention on par with, if not better than, comparative sunscreen formulations.

**Example 6**

***Volatile Aromatic Odor of Exemplary and Comparative Sunscreen Formulations***

The odor profile of exemplary and comparative particles as prepared in Example 1 was assessed by the total aromatic level within the particles. To analyze the sample for volatile aromatics, the samples were run on an Agilent 6890 GC with 5973 MS detector and a Perkin Elmer TurboMatrix 40 Trap headspace autosampler. A number of aromatics commonly found in the headspace volatiles of latex binders (toluene, styrene, propylbenzene, benzaldehyde) were calibrated using a set of standards of known concentrations. The calibration standards were first prepared with known weight concentration, 1-1000 ppm wt/wt in THF. The standards were prepared by weighing 10-15 mg of each calibration mix into 22 mL headspace vials and capping with Teflon-lined septa. The headspace analysis of the standards was done in a full-evaporation mode to eliminate matrix effects that can occur in static headspace sampling. In this mode, a small sample size is used, and the headspace vial temperature is set sufficiently high to allow for full evaporation of the volatile of interest. For this analysis, the standard samples were heated to 150°C for 10 minutes prior to sampling. A calibration plot was prepared for each volatile of interest using at least three standard concentrations for that compound. The mg amount of each compound in each sample was then determined using the linear-least-squares equation from the calibration plot for that compound. The ppm (wt/wt) concentration of each compound in each sample was then determined by dividing the mg amount of each compound by the initial weight of the sample and then multiplying by 1,000,000. An average response factor was used to calibrate any compound in the sample that did not have a calibration standard. The individual concentrations of all observed aromatic species were then summed to obtain the total aromatic concentration in the sample (ppm, wt/wt). Water and air blanks were run before and after the sequence. The mass spectrums of the analytes were used to distinguish any co-eluting compounds based on their characteristic ions. All samples were run at least three times to ensure reproducibility. The results of the aromatic analysis are shown in Table 7.

**Table 7. Total Volatile Aromatic Level**

| Sample | Total Aromatic (ppm) |
|---|---|
| P-C1* | 615 |
| P-C2* | 435 |
| P-E1 | 84 |
| P-E2 | 65 |
| P-E3 | 28 |
| P-E4 | 31 |
| P-E5 | 64 |
| P-E6 | 68 |
| *Comparative | |

The results of the volatile aromatic odor study demonstrate that exemplary sunscreen formulations prepared according to the invention provide an odor profile that is on par with, if not better than, comparative particles.

**Claims**

1. A personal care composition comprising:

   (A) voided latex particles comprising

   (i) at least one core polymer comprising polymerized units derived from (a) 20 to 60 weight % of monoethylenically unsaturated monomers containing at least one carboxylic acid group, based on the total weight of the core polymer, and (b) 40 to 80 weight % of non-ionic ethylenically unsaturated monomers, based on the total weight of the core polymer; and
   (ii) at least one shell polymer comprising polymerized units derived from (a) 10 to 70 weight % of non-ionic ethylenically unsaturated monomers, based on the total weight of the shell polymer(s), and (b) 30 to 90 weight % of aliphatic monomers selected from the group consisting of allyl methacrylate, trimethylolpropane trimethacrylate, trimethylolpropane triacrylate, and combinations thereof, based on the total weight of the shell polymer(s); and

   (B) at least one UV absorbing agent,

   wherein the voided latex particles are present in an amount of from 0.5 to 20 weight %, based on the total weight of the composition, and wherein the voided latex particles contain a void and have a particle size of from 100 nm to 400 nm; and wherein an outermost shell comprises a shell polymer having polymerized units of the aliphatic monomers in an amount from 40 to 80 weight % based on the weight of the outermost shell polymer.

2. The personal care composition of claim 1, wherein the aliphatic monomers of the at least one shell polymer(s) comprise one or more tri(meth)acrylates selected from the group consisting of trimethylolpropane trimethacrylate, trimethylolpropane triacrylate, 1,2,4-butanetriol triacrylate, 1,2,4-butanetriol trimethacrylate, glycerol triacrylate, lycerol trimethacrylate, pentaerythritol triacrylate, pentaerythritol trimethacrylate, polyoxypropyltrimethylolpropane triacrylate, polyoxypropyltrimethylolpropane trimethacrylate, silicone triacrylate, silicone trimethacrylate, 1,3,5-triacryloylhexahydro-s-triazine, 1,3,5-trimethacryloylhexahydro-s-triazine, trimethylolethane triacrylate, trimethylolethane trimethacrylate, 1,1,1-trimethylol propane triacrylate, 1,1,1-trimethylol propane trimethacrylate, 1,2,3-trimethylolpropane triacrylate, 1,2,3-trimethylol propane trimethacrylate, 1,1,1-trimethylol propane tris(acryloxypropionate), 1,1,1-trimethylol propane tris(methacryloxypropionate), 1,2,3-trimethylol propane tris(acryloxypropionate), 1,2,3-trimethylol propane tris(methacryloxypropionate), tris-(2-acryloxyethyl) isocyanurate, tris-(2-methacryloxyethyl) isocyanurate, and mixtures thereof.

3. The personal care composition of claim 1, wherein the aliphatic monomers of the at least one shell polymer(s) comprise one or more (meth)acrylic monomers having mixed ethylenic functionality selected from the group consisting of the acrylate ester of neopentyl glycol monodicyclopentenyl ether, allyl acryloxypropionate, allyl acrylate, allyl methacrylate, crotyl acrylate, crotyl methacrylate, 3-cyclohexenylmethyleneoxyethyl acrylate, 3-cyclohexenylmethyleneoxyethyl methacrylate, dicyclopentadienyloxyethyl acrylate, dicyclopentadienyloxyethyl methacrylate, dicyclopentenyl acrylate, dicyclopentenyl methacrylate, dicyclopentenyloxyethyl acrylate, dicycol pentenyloxyethyl methacrylate, methacrylate ester of neopentyl glycol monodicyclopentenyl ether, methallyl acrylate, trimethylolpropane diallyl ether mono-acrylate, trimethylolpropane diallyl ether mono-methacrylate, N-allyl acrylamide, and mixtures thereof.

4. The composition of claim 1, wherein the non-ionic ethylenically unsaturated monomers of the at least one shell are selected from the group consisting of styrene, ethylvinylbenzene, t-butylstrene, and combinations thereof.

5. The composition of claim 1, wherein
the monoethylenically unsaturated monomers containing at least one carboxylic acid group of the core polymer comprise a monomer selected from the group consisting of (meth)acrylic acid, (meth)acryloxypropionic acid, itaconic acid, aconitic acid, maleic acid, maleic anhydride, fumaric acid, cronotic acid, citraconic acid, maleic anhydride, monomethyl maleate, monomethyl fumarate, monomethyl itaconate, and mixtures thereof, and
the non-ionic ethylenically unsaturated monomers of the core polymer comprise a monomer selected from the group consisting of styrene, vinyltoluene, ethylene, vinyl acetate, vinyl chloride, vinylidene chloride acrylonitrile, (meth)acrylamide, methyl (meth)acrylate, ethyl (meth)acrylate, butyl (meth)acrylate, 2-ethylhexyl (meth)acrylate, benzyl (meth)acrylate, lauryl (meth)acrylate, oleyl (meth)acrylate, palmityl (meth)acrylate, stearyl (meth)acrylate, and mixtures thereof.

6. The personal care composition of claim 1, wherein the at least one shell polymer further comprises polymerized units derived from 0.1 to 5 weight % of a monoethylenically unsaturated monomer containing at least one carboxylic acid group.

7. The personal care composition of claim 1, wherein the at least one shell polymer further comprises polymerized units derived from 0.1 to 5 weight % of a monoethylenically unsaturated monomer containing at least one "non-carboxylic" acid group.

8. A personal care composition according to claim 1 for use in a method for protecting skin from UV damage, the method comprising topically administering to the skin an effective amount of the personal care composition.

9. A personal care composition according to claim 1 for use in a method for boosting the SPF or UV absorption of a sunscreen composition comprising adding to said sunscreen composition from 0.5 to 20 weight % of voided latex particles, based on the total weight of the composition.

**Patentansprüche**

1. Eine Körperpflegezusammensetzung, die Folgendes beinhaltet:

(A) Latexteilchen mit Hohlräumen, die Folgendes beinhalten:

(i) mindestens ein Kernpolymer, das polymerisierte Einheiten beinhaltet, die (a) zu 20 bis 60 Gew.-%, bezogen auf das Gesamtgewicht des Kernpolymers, von monoethylenisch ungesättigten Monomeren, die mindestens eine Carbonsäuregruppe enthalten, und (b) zu 40 bis 80 Gew.-%, bezogen auf das Gesamtgewicht des Kernpolymers, von nichtionischen ethylenisch ungesättigten Monomeren abgeleitet sind; und
(ii) mindestens ein Schalenpolymer, das polymerisierte Einheiten beinhaltet, die (a) zu 10 bis 70 Gew.-%, bezogen auf das Gesamtgewicht des Schalenpolymers/der Schalenpolymere, von nichtionischen ethylenisch ungesättigten Monomeren und (b) zu 30 bis 90 Gew.-%, bezogen auf das Gesamtgewicht des Schalenpolymers/der Schalenpolymere, von aliphatischen Monomeren, die aus der Gruppe ausgewählt sind, die aus Allylmethacrylat, Trimethylolpropantrimethacrylat, Trimethylolpropantriacrylat und Kombinationen davon besteht, abgeleitet sind; und

(B) mindestens ein UV-Absorptionsmittel,

wobei die Latexteilchen mit Hohlräumen in einer Menge von 0,5 bis 20 Gew.-%, bezogen auf das Gesamtgewicht der Zusammensetzung, vorhanden sind und wobei die Latexteilchen mit Hohlräumen einen Hohlraum enthalten und eine Partikelgröße von 100 nm bis 400 nm aufweisen; und wobei eine äußerste Schale ein Schalenpolymer beinhaltet, das polymerisierte Einheiten der aliphatischen Monomere in einer Menge von 40 bis 80 Gew.-%, bezogen auf das Gewicht des äußersten Schalenpolymers, aufweist.

2. Körperpflegezusammensetzung gemäß Anspruch 1, wobei die aliphatischen Monomere des mindestens einen Schalenpolymers ein oder mehrere Tri(meth)acrylate beinhalten, die aus der Gruppe ausgewählt sind, die aus Folgendem besteht:

Trimethylolpropantrimethacrylat, Trimethylolpropantriacrylat, 1,2,4-Butantrioltriacrylat, 1,2,4-Butantrioltrimethacrylat, Glyceroltriacrylat, Lyceroltrimethacrylat, Pentaerythritoltriacrylat, Pentaerythritoltrimethacrylat, Polyoxypropyltrimethylolpropantriacrylat, Polyoxypropyltrimethylolpropantrimethacrylat, Silicontriacrylat, Silicontrimethacrylat, 1,3,5-Triacryloylhexahydro-s-triazin, 1,3,5-Trimethacryloylhexahydro-s-triazin, Trimethylolethantriacrylat, Trimethylolethantrimethacrylat, 1,1,1-Trimethylolpropantriacrylat, 1,1,1-Trimethylolpropantrimethacrylat, 1,2,3-Trimethylolpropantriacrylat, 1,2,3-Trimethylolpropantrimethacrylat, 1,1,1-Trimethylolpropantris(acryloxypropionat), 1,1,1-Trimethylolpropantris(methacryloxypropionat), 1,2,3-Trimethylolpropantris(acryloxypropionat), 1,2,3-Trimethylolpropantris(methacryloxypropionat), Tris(2-acryloxyethyl)isocyanurat, Tris(2-methacryloxyethyl)isocyanurat und Mischungen davon.

3. Körperpflegezusammensetzung gemäß Anspruch 1, wobei die aliphatischen Monomere des mindestens einen Schalenpolymers ein oder mehrere (Meth)acrylmonomere beinhalten, die gemischte Ethylenfunktionalität aufweisen und aus der Gruppe ausgewählt sind, die aus Folgendem besteht: dem Acrylatester von Neopentylglycolmonodicyclopentenylether, Allylacryloxypropionat, Allylacrylat, Allylmethacrylat, Crotylacrylat, Crotylmethacrylat, 3-Cyclohexenylmethylenoxyethylacrylat, 3-Cyclohexenylmethylenoxyethylmethacrylat, Dicyclopentadienyloxyethylacrylat, Dicyclopentadienyloxyethylmethacrylat, Dicyclopentenylacrylat, Dicyclopentenylmethacrylat, Dicyclopentenyloxyethylacrylat, Dicyclopentenyloxyethylmethacrylat, Methacrylatester von Neopentylglycolmonodicyclopentenylether, Methallylacrylat, Trimethylolpropandiallylethermonoacrylat, Trimethylolpropandiallylethermonomethacrylat, N-Allylacrylamid und Mischungen davon.

4. Zusammensetzung gemäß Anspruch 1, wobei die nichtionischen ethylenisch ungesättigten Monomere der mindestens einen Schale aus der Gruppe ausgewählt sind, die aus Styrol, Ethylvinylbenzol, t-Butylstyrol und Kombinationen davon besteht.

5. Zusammensetzung gemäß Anspruch 1, wobei:

die monoethylenisch ungesättigten Monomere des Kernpolymers, die mindestens eine Carbonsäuregruppe enthalten, ein Monomer beinhalten, das aus der Gruppe ausgewählt ist, die aus (Meth)acrylsäure, (Meth)acryloxypropionsäure, Itaconsäure, Aconitsäure, Maleinsäure, Maleinsäureanhydrid, Fumarsäure, Crotonsäure, Citraconsäure, Maleinsäureanhydrid, Monomethylmaleat, Monomethylfumarat, Monomethylitaconat und Mischungen davon besteht, und
die nichtionischen ethylenisch ungesättigten Monomere des Kernpolymers ein Monomer beinhalten, das aus der Gruppe ausgewählt ist, die aus Styrol, Vinyltoluol, Ethylen, Vinylacetat, Vinylchlorid, Vinylidenchloridacrylonitril, (Meth)acrylamid, Methyl(meth)acrylat, Ethyl(meth)acrylat, Butyl(meth)acrylat, 2-Ethylhexyl(meth)acrylat, Benzyl(meth)acrylat, Lauryl(meth)acrylat, Oleyl(meth)acrylat, Palmityl(meth)acrylat, Stearyl(meth)acrylat und Mischungen davon besteht.

6. Körperpflegezusammensetzung gemäß Anspruch 1, wobei das mindestens eine Schalenpolymer ferner polymerisierte Einheiten beinhaltet, die zu 0,1 bis 5 Gew.-% von einem monoethylenisch ungesättigten Monomer abgeleitet sind, das mindestens eine Carbonsäuregruppe enthält.

7. Körperpflegezusammensetzung gemäß Anspruch 1, wobei das mindestens eine Schalenpolymer ferner polymerisierte Einheiten beinhaltet, die zu 0,1 bis 5 Gew.-% von einem monoethylenisch ungesättigten Monomer abgeleitet sind, das mindestens eine "Nichtcarbonsäure"-Gruppe enthält.

8. Körperpflegezusammensetzung gemäß Anspruch 1 zur Verwendung in einem Verfahren zum Schutz von Haut vor UV-Schaden, wobei das Verfahren das topische Applizieren einer effektiven Menge der Körperpflegezusammensetzung auf die Haut beinhaltet.

9. Körperpflegezusammensetzung gemäß Anspruch 1 zur Verwendung in einem Verfahren zum Erhöhen des LSF oder der UV-Absorption einer Sonnenschutzzusammensetzung, das das Hinzufügen zu der Sonnenschutzzusammensetzung von Latexteilchen mit Hohlräumen zu 0,5 bis 20 Gew.-%, bezogen auf das Gesamtgewicht der Zusammensetzung, beinhaltet.

**Revendications**

1. Une composition pour soins personnels comprenant :

   (A) des particules de latex à vide comprenant

       (i) au moins un polymère de cœur comprenant des unités polymérisées dérivées (a) de 20 à 60 % en poids de monomères monoéthyléniquement insaturés contenant au moins un groupe acide carboxylique, rapporté au poids total du polymère de cœur, et (b) de 40 à 80 % en poids de monomères éthyléniquement insaturés non ioniques, rapporté au poids total du polymère de cœur ; et
       (ii) au moins un polymère d'enveloppe comprenant des unités polymérisées dérivées (a) de 10 à 70 % en poids de monomères éthyléniquement insaturés non ioniques, rapporté au poids total du/des polymère(s) d'enveloppe, et (b) de 30 à 90 % en poids de monomères aliphatiques sélectionnés dans le groupe constitué de méthacrylate d'allyle, de triméthacrylate de triméthylolpropane, de triacrylate de triméthylolpropane, et de combinaisons de ceux-ci, rapporté au poids total du/des polymère(s) d'enveloppe ; et

   (B) au moins un agent absorbant les UV,

   dans laquelle les particules de latex à vide sont présentes en une quantité allant de 0,5 à 20 % en poids, rapporté au poids total de la composition, et dans laquelle les particules de latex à vide contiennent un vide et ont une taille de particule allant de 100 nm à 400 nm ; et dans laquelle une enveloppe la plus externe comprend un polymère d'enveloppe ayant des unités polymérisées des monomères aliphatiques en une quantité allant de 40 à 80 % en poids rapporté au poids du polymère d'enveloppe la plus externe.

2. La composition pour soins personnels de la revendication 1, dans laquelle les monomères aliphatiques de l'au moins un polymère d'enveloppe comprennent un ou plusieurs tri(méth)acrylates sélectionnés dans le groupe constitué de triméthacrylate de triméthylolpropane, de triacrylate de triméthylolpropane, de triacrylate de 1,2,4-butanetriol, de triméthacrylate de 1,2,4-butanetriol, de triacrylate de glycérol, de triméthacrylate de lycérol, de triacrylate de pentaérythritol, de triméthacrylate de pentaérythritol, de triacrylate de polyoxypropyltriméthylolpropane, de triméthacrylate de polyoxypropyltriméthylolpropane, de triacrylate de silicone, de triméthacrylate de silicone, de 1,3,5-triacryloylhexahydro-s-triazine, de 1,3,5-triméthacryloylhexahydro-s-triazine, de triacrylate de triméthyloléthane, de triméthacrylate de triméthyloléthane, de triacrylate de 1,1,1-triméthylolpropane, de triméthacrylate de 1,1,1-triméthylolpropane, de triacrylate de 1,2,3-triméthylolpropane, de triméthacrylate de 1,2,3-triméthylolpropane, de tris(acryloxypropionate) de 1,1,1-triméthylolpropane, de tris(méthacryloxypropionate) de 1,1,1-triméthylolpropane, de tris(acryloxypropionate) de 1,2,3-triméthylolpropane, de tris(méthacryloxypropionate) de 1,2,3-triméthylolpropane, d'isocyanurate de tris-(2-acryloxyéthyle), d'isocyanurate de tris-(2-méthacryloxyéthyle), et de mélanges de ceux-ci.

3. La composition pour soins personnels de la revendication 1, dans laquelle les monomères aliphatiques de l'au moins un polymère d'enveloppe comprennent un ou plusieurs monomères (méth)acryliques ayant une fonctionnalité éthylénique mixte sélectionnés dans le groupe constitué de l'ester acrylate d'éther monodicyclopenténylique de néopentylglycol, d'acryloxypropionate d'allyle, d'acrylate d'allyle, de méthacrylate d'allyle, d'acrylate de crotyle, de méthacrylate de crotyle, d'acrylate de 3-cyclohexénylméthylèneoxyéthyle, de méthacrylate de 3-cyclohexénylméthylèneoxyéthyle, d'acrylate de dicyclopentadiényloxyéthyle, de méthacrylate de dicyclopentadiényloxyéthyle, d'acrylate de dicyclopentényle, de méthacrylate de dicyclopentényle, d'acrylate de dicyclopentényloxyéthyle, de méthacrylate de dicyclopentényloxyéthyle, de l'ester méthacrylate d'éther monodicyclopenténylique de néopentylglycol, d'acrylate de méthallyle, de mono-acrylate d'éther diallylique de triméthylolpropane, de mono-méthacrylate d'éther diallylique de triméthylolpropane, de N-allylacrylamide, et de mélanges de ceux-ci.

4. La composition de la revendication 1, dans laquelle les monomères éthyléniquement insaturés non ioniques de l'au moins une enveloppe sont sélectionnés dans le groupe constitué de styrène, d'éthylvinylbenzène, de t-butylstyrène, et de combinaisons de ceux-ci.

5. La composition de la revendication 1, dans laquelle
les monomères monoéthyléniquement insaturés contenant au moins un groupe acide carboxylique du polymère de cœur comprennent un monomère sélectionné dans le groupe constitué d'acide (méth)acrylique, d'acide (méth)acryloxypropionique, d'acide itaconique, d'acide aconitique, d'acide maléique, d'anhydride maléique, d'acide fumarique, d'acide crotonique, d'acide citraconique, d'anhydride maléique, de maléate de monométhyle, de fumarate de monométhyle, d'itaconate de monométhyle, et de mélanges de ceux-ci, et
les monomères éthyléniquement insaturés non ioniques du polymère de cœur comprennent un monomère sélectionné dans le groupe constitué de styrène, de vinyltoluène, d'éthylène, d'acétate de vinyle, de chlorure de vinyle, d'acrylonitrile de chlorure de vinylidène, de (méth)acrylamide, de (méth)acrylate de méthyle, de (méth)acrylate d'éthyle, de (méth)acrylate de butyle, de (méth)acrylate de 2-éthylhexyle, de (méth)acrylate de benzyle, de (méth)acrylate de lauryle, de (méth)acrylate d'oléyle, de (méth)acrylate de palmityle, de (méth)acrylate de stéaryle, et de mélanges de ceux-ci.

6. La composition pour soins personnels de la revendication 1, dans laquelle l'au moins un polymère d'enveloppe comprend en outre des unités polymérisées dérivées de 0,1 à 5 % en poids d'un monomère monoéthyléniquement insaturé contenant au moins un groupe acide carboxylique.

7. La composition pour soins personnels de la revendication 1, dans laquelle l'au moins un polymère d'enveloppe comprend en outre des unités polymérisées dérivées de 0,1 à 5 % en poids d'un monomère monoéthyléniquement insaturé contenant au moins un groupe acide « non carboxylique ».

8. Une composition pour soins personnels selon la revendication 1 pour une utilisation dans un procédé pour la protection de la peau contre les méfaits des UV, le procédé comprenant l'administration topique sur la peau d'une quantité efficace de la composition pour soins personnels.

9. Une composition pour soins personnels selon la revendication 1 pour une utilisation dans un procédé pour le renforcement du FPS ou de l'absorption UV d'une composition d'écran solaire comprenant l'ajout à ladite composition d'écran solaire de 0,5 à 20 % en poids de particules de latex à vide, rapporté au poids total de la composition.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5663213 A

**Non-patent literature cited in the description**

- *Bulletin of the American Physical Society,* 1956, vol. 1 (3), 123

- Polymer Handbook. Interscience Publishers